(19) Europäisches Patentamt
European Patent Office
Office européen des brevets

(11) Veröffentlichungsnummer: **0 320 757**
**A2**

(12) # EUROPÄISCHE PATENTANMELDUNG

(21) Anmeldenummer: **88120337.6**

(22) Anmeldetag: **06.12.88**

(51) Int. Cl.4: **C08F 220/34 , A61L 25/00 , A61F 5/445 , //(C08F220/34, 220:12)**

(30) Priorität: **15.12.87 DE 3742472**

(43) Veröffentlichungstag der Anmeldung:
**21.06.89 Patentblatt 89/25**

(84) Benannte Vertragsstaaten:
**AT BE CH DE ES FR GB IT LI LU NL SE**

(71) Anmelder: **BELLAND AG**
**Sandmattstrasse 2**
**CH-4500 Solothurn(CH)**

(72) Erfinder: **Deibig, Heinrich, Dr.**
**Vorstettli 395**
**CH-4524 Günsberg(CH)**
Erfinder: **Dinkelaker, Albrecht**
**Blümlisalpstr. 20**
**CH-4562 Biberist(CH)**

(74) Vertreter: **Patentanwälte Ruff und Beier**
**Neckarstrasse 50**
**D-7000 Stuttgart 1(DE)**

(54) **Aminogruppen enthaltendes Polymerisat, Verfahren zu seiner Herstellung und Verwendung.**

(57) Die Erfindung betrifft Aminogruppen enthaltende Polymerisate, die durch Polymerisation von Acrylaten mit Aminoacrylaten in Form von Estern von Acrylsäure oder Methacrylsäure mit Aminoalkoholen hergestellt sind. Polymere dieser Art sind in neutralem bis basischem wäßrigem Medium unlöslich aber in saurem Medium auflösbar. Sie eignen sich besonders für die Herstellung von Colostomiebeuteln.

EP 0 320 757 A2

## Aminogruppen enthaltendes Polymerisat, Verfahren zu seiner Herstellung und Verwendung

In der europäischen Patentschrift 32 244 ist eine Verbundfolie beschrieben, bei der eine Schicht aus einem in Wasser nicht löslichen aber in saurem oder basischem wäßrigen Medium lösbaren Material besteht. Bei dem Material handelt es sich vorzugsweise um ein Copolymerisat einer ungesättigten organischen Carbonsäure mit einem neutralen Monomeren.

In der europäischen Offenlegungsschrift 143 935 sind in Wasser unlösliche aber in basischem Medium auflösbare Copolymerisate aus Acrylat und Acrylsäure bzw. Methacrylsäure beschrieben, die vielseitig verwendbar sind und gute mechanische Eigenschaften besitzen.

Aufgabe der Erfindung ist es, Polymerisate zur Verfügung zu stellen, die in neutralem und basischem Medium unlöslich sind, aber in sauren, wäßrigen Medien aufgelöst werden können.

Gegenstand der Erfindung sind deshalb Aminogruppen enthaltende Polymerisate aus mindestens einem Acrylat der Formel I

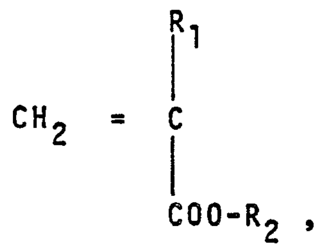

I

bei der $R_1$ Wasserstoff oder Methyl und $R_2$ ein Alkylrest mit 1 bis 18 C-Atomen sind, und einem Aminoacrylat der Formel II

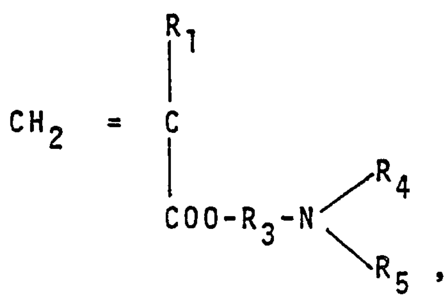

II

bei der $R_1$ dieselbe Bedeutung hat wie oben und $R_3$ ein Alkylrest mit 1 bis 6 C-Atomen ist und $R_4$ und $R_5$ gleich oder verschieden sind und Wasserstoff oder Alkylreste mit 1 bis 4 C-Atomen sind, sowie gegebenenfalls neutralen Vinylmonomeren, die von den Acrylaten der Formel I verschieden sind, und üblichen Polymerisatzusätzen.

Polymerisate mit der obengenannten Zusammensetzung stellen eine wertvolle Ergänzung der bekannten, in basischen Medien löslichen Polymerisate dar und eröffnen zahlreiche neue Anwendungsmöglichkeiten.

Der Rest $R_2$ der Monomere der Formel I ist vorzugsweise ein Alkylrest mit 1 bis 4 C-Atomen. Typische Vertreter für die Monomere der Formel I sind Methylmethacrylat, Ethylacrylat, Butylacrylat und Butylmethacrylat. Bei den Aminoacrylaten der Formel II ist der Rest $R_3$ vorzugsweise C2. Die Reste $R_4$ und $R_5$ sind vorzugsweise Alkylreste, insbesondere Methyl oder Ethyl. Das Stickstoffatom kann sich weiterhin innerhalb eines anstelle der Reste $R_4$ und $R_5$ vorgesehenen Ringes, wie beispielsweise eines Morpholinringes, befinden. Ein besonders geeigneter Vertreter der Aminoacrylate ist Dimethylaminoethylmethacrylat (DIMA).

Das Termonomer kann ein Monomer der Gruppe Vinyläther mit 3 bis 18 C-Atomen, insbesondere Vinylisobutyläther, aromatische Vinylkohlenwasserstoffe mit 8 bis 11 C-Atomen, insbesondere Styrol, Acrylat bzw. Methacrylat mit einem Alkoholrest mit 1 bis 18 C-Atomen, insbesondere n-Butylacrylat, Butylmethacrylat und Stearylmethacrylat, sein.

Das Molverhältnis der Monomere der Formel I zu den Monomeren der Formel II liegt vorzugsweise im Bereich von 8 : 1 bis 2 : 1, insbesondere 6 : 1 bis 3 : 1. Das Verhältnis von Aminoacrylat zu Terpolymer

liegt, sofern ein solches vorhanden ist, vorzugsweise bei 1 : 0, 1 bis 1 : 1, insbesondere 1 : 0, 2 bis 1 : 0,5. Das Molekulargewicht der Polymere liegt vorzugsweise im Bereich von 40.000 bis 200.000, insbesondere 50.000 bis 80.000.

Zur Herstellung der erfindungsgemäßen Polymerisate werden die Monomere vorzugsweise direkt in Form des Acrylats bzw. Aminoacrylats polymerisiert. Die Polymerisation kann kontinuierlich durchgeführt werden. Hierzu eignen sich wiederum besonders Extruder, insbesondere Anlagen mit mehreren in Reihe geschalteten Extrudern. Die Polymere können in verschiedenen Zustandsformen vorliegen, zum Beispiel in Form von Lösungen oder Dispersionen, die für Verklebungen oder Beschichtungen verwendbar sind, sowie auch als Pulver. Aufgrund ihrer thermoplastischen Eigenschaften können sie auch in Form von Formkörpern, insbesondere Granulaten, Folien, Tiefziehteilen, Spritzgußteilen, Preß- oder ßlasteilen sowie Platten, vorliegen. Diese Formkörper können mit sauer wirkenden Überzügen versehen sein, um sie von der mit den Überzügen versehenen Seite her wasserlösbar zu machen.

Die erfindungsgemäßen Polymere eignen sich besonders für die Verwendung bei der Herstellung von auflösbaren Behältnissen, die zur Aufnahme von Kot bestimmt sind, insbesondere zur Herstellung von sogenannten Colostomiebeuteln.

Colostomiebeutel werden zum Auffangen von Kot bei künstlichen Darmausgängen verwendet. Ihre Beseitigung nach Gebrauch stellt ein Problem dar, weil die hier verwendeten Folien mechanisch und chemisch außerordentlich stabil sind. Da Kot einen neutralen bis basischen pH-Wert besitzt, eignen sich für die innere Schicht solcher Auffangbehältnisse besonders Kunststoffe, die zwar im neutralen bis basischen Bereich unlöslich sind, jedoch im sauren Bereich aufgelöst werden können. Dies ist bei den erfindungsgemäßen Polymerisaten, wie gesagt, der Fall.

Die Colostomiebeutel bzw. sonstigen Behältnisse können aus einer Folie des in basischem und neutralem Bereich stabilen Kunststoffes bestehen und dann nach Gebrauch durch Eintauchen oder sonstiges Zusammenbringen mit saurem Material lösbar gemacht werden. Bevorzugt sind jedoch Systeme, in die die Säure bereits eingebaut ist, so daß sie auch in neutralem wäßrigem Milieu lösbar sind und eine Säure nicht gesondert bereitgehalten werden muß. Hierzu kann eine Folie als Verbundfolie mit mindestens zwei Schichten ausgebildet sein, wobei eine an der Außenseite der säurelöslichen, aber in neutralem bzw. basischem Medium stabilen Innenschicht angrenzende Schicht ihrerseits aus saurem Material bestehen oder ein solches enthalten kann. Dabei ist diese saure Schicht vorzugsweise ihrerseits wasserlöslich, so daß sie beim Auflösen die Säure freisetzt, die dann als Lösungsvermittler für die in saurem Medium lösbare Innenschicht frei setzt. Die Verbundfolie kann zusätzlich noch eine Dampf-bzw. Geruchssperre aufweisen. Hierzu eignet sich eine Aluminiumfolie bzw. -beschichtung. Dabei kann die Aluminiumschicht ihrerseits so dünn gehalten werden, daß sie bei der Auflösung der Folien zerfällt. Die Aluminiumschicht kann jedoch auch zwischen Folienschichten eingelagert werden, so zum Beispiel zwischen die erwähnte saure Schicht und eine äußere wasserlösliche Schicht, in die Stoffe eingelagert sein können, die bei Wasserzutritt eine Zerstörung der Aluminiumschicht bewirken, wie Alkalien oder oxidativ wirkende Substanzen. Schließlich kann der Beutel bzw. das Behältnis an seiner Außenseite mindestens eine hautfreundliche Schutzschicht aufweisen, die an ihrer Außenseite mit einer textilen Beflockung oder dergleichen versehen sein kann. Dadurch kann ein für den Träger des Beutels unangenehmes Schwitzen und auch ein ungewollter Anlösevorgang durch Schwitzen vermieden werden.

Weitere Merkmale und Vorteile der Erfindung ergeben sich aus den nachfolgenden Beispielen in Verbindung mit den Unteransprüchen. Hierbei können die einzelnen Merkmale jeweils für sich allein oder in Kombination miteinander verwirklicht sein.

## Beispiel 1

In einem auf 20 °C gekühlten doppelwandigen Rührbehälter wurden 60 Mol (51,6 kg) Methylacrylat, 10 Mol (15,7 kg) Dimethylaminoethylmethacrylat (im folgenden als "DIMA" bezeichnet) und 0,5 kg Azobisdiisobutyronitril (im folgenden abgekürzt mit "AIBN") gelöst und diese Reaktionsmischung mit einer Rate von 10 kg/h in die Einfüllzone eines gleichsinnig drehenden dichtkämmenden Zweischneckenextruders von 60 mm Durchmesser und 2580 mm Länge gepumpt.

Der Extruder, der im folgenden als Reaktor bezeichnet wird, hat zehn temperierbare (heiz- und kühlbare) Zonen. Die Einpumpzone hatte eine Temperatur von 80 C°, die Zonen 2 bis 10 waren auf 105 °C temperiert. Bei einer Reaktordrehzahl von 30/min wurde eine Stromaufnahme von 9 A gemessen; der Druck am Reaktorende betrug 8 bar.

Das Polymerisationsgemisch wurde aus dem Reaktor über eine Schmelzeleitung in einen zweiten

gleichsinnig drehenden dichtkämmenden Zweischneckenextruder von 30 mm Durchmesser und 1300 mm Länge gefördert. Dieser Extruder wird im folgenden als Compounder bezeichnet.

Der Compounder hat sechs heiz- und kühlbare Zonen. Zone 2 (vom Reaktor her betrachtet) trägt einen Destillationsaufsatz, die Zonen 3 und 4 sind mit Vakuumpumpen zur Entfernung von restlichen Monomeren und Lösungsmitteln verbunden. Die Compoundertemperaturen betrugen 140 °C in Zone 1, 180 °C in den Zonen 2, 3 und 4 und 120 °C in Zone 6 (Extruderkopf, Düsen).

Aus dem Reaktor-Polymerisationsgemisch wurden 0,6 kg/h nicht polymerisierte Monomere abdestilliert; die Stromaufnahme im Compounder betrug 20 A. Das Polymerisat verließ den Compounder in Form von Strängen, die auf Kühlwalzen abgekühlt und in einem Granulator granuliert wurden.

Das Granulat besaß einen analytisch bestimmten (Kjeldahl) Stickstoffgehalt von 2,6 % (theoretisch 2,1 %). Aus dem Granulat lassen sich transparente Folien herstellen, die gegen neutrales und basisches Wasser beständig sind, sich aber in verdünnten Säuren auflösen.

Die Eigenschaft der Wasserunlöslichkeit und der Löslichkeit in Säuren macht die Produkte sehr geeignet für temporäre Schutz- und Abdeckfunktionen sowie als intermediäre Hilfsmittel in Verarbeitungsprozessen.

## Beispiel 2

Verwendet man anstelle der Ansätze des Beispiels 1 die folgenden
50 Mol (50 kg) Ethylacrylat
10 Mol (15,7 kg) DIMA
0,5 kg AIBN,
so erhält man ein Copolymerisat mit 2,5 % N (theoretisch 2,1 % N) und mit ähnlichen Eigenschaften.

## Beispiel 3

Verwendet man anstelle der Ansätze des Beispiels 1 die folgenden
40 Mol (56,8 kg) Butylmethacrylat
10 Mol (15,7 kg) DIMA
0,5 kg AIBN,
so erhält man ein Copolymerisat mit 2,4 % N (theoretisch 1,9 % N) und mit ähnlichen Eigenschaften.

**Ansprüche**

1. Aminogruppen enthaltendes Polymerisat aus mindestens einem Acrylat der Formel I

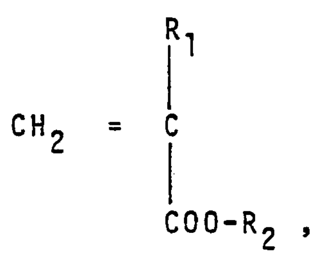

I

bei der $R_1$ Wasserstoff oder Methyl und $R_2$ ein Alkylrest mit 1 bis 18 C-Atomen sind und einem Aminoacrylat der Formel II

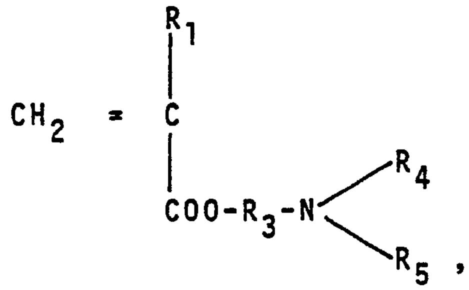

II

bei der $R_1$ dieselbe Bedeutung hat wie oben, $R_3$ ein Alkylrest mit 1 bis 6 C-Atomen ist und $R_4$ und $R_5$ gleich oder verschieden sind und Wasserstoff oder Alkylreste mit 1 bis 4 C-Atomen sind, sowie gegebenenfalls neutralen Termonomeren, die vom Monomer der Formel I verschieden sind, und üblichen Polymerisationszusätzen.

2. Polymerisat nach Anspruch 1, dadurch gekennzeichnet, daß das Termonomere ein Monomer der Gruppe Vinyläther mit 3 bis 18 C-Atomen, insbesondere Vinylisobutyläther, aromatische Vinylkohlenwasserstoffe mit 8 bis 11 C-Atomen, insbesondere Styrol, Acrylat bzw. Methacrylat mit einem Alkoholrest mit 1 bis 18 C-Atomen, insbesondere n-Butylacrylat, Butylmethacrylat und Stearylmethacrylat, ist.

3. Polymerisat nach Anspruch 1 oder 2, dadurch gekennzeichnet, daß das Molverhältnis des Monomers der Formel I zum Monomer der Formel II bei 8 : 1 bis 2 : 1, insbesondere 6 : 1 bis 3,5 : 1 liegt.

4. Polymerisat nach einem der Ansprüche 1 bis 3, dadurch gekennzeichnet, daß das Termonomere in Mengen von 0,1 bis 1, insbesondere 0,1 bis 0,4 Mol/Mol Monomer der Formel II vorliegt.

5. Polymerisat nach einem der vorhergehenden Ansprüche in Form eines Granulats, einer Folie, eines Tiefziehteils, Spritzgußteils, Preß- oder Blasteils, gegebenenfalls in Verbindung mit sauer wirkenden wasserlöslichen Überzügen.

6. Verwendung des Polymerisats nach einem der vorhergehenden Ansprüche, als im neutralen und alkalischen pH-Bereich unlösliche, aber im sauren pH-Bereich auflösbare Innenschicht von auflösbaren Fäkalienbehältnissen, insbesondere Colostomiebeuteln.

7. Verwendung des Polymerisats nach Anspruch 6 in Form einer Folie, insbesondere einer Verbundfolie mit mindestens zwei Schichten.

8. Verwendung nach Anspruch 6 oder 7, dadurch gekennzeichnet, daß das Behältnis von außen her in Wasser auflösbar ist, insbesondere eine an der Außenseite der Innenschicht angrenzende wasserlösliche Schicht aus saurem Material besteht oder ein sauer wirkendes Mittel enthält.

9. Verfahren zur Herstellung des Polymerisats nach einem der Ansprüche 1 bis 5, durch Polymerisation eines Monomers der Formel I mit einem Monomer der Formel II, gegebenenfalls in Gegenwart von Termonomeren und üblichen Polymerisationszusätzen.

10. Verfahren nach Anspruch 9, dadurch gekennzeichnet, daß die Polymerisation kontinuierlich unter Verwendung mindestens eines Extruders durchgeführt wird, indem die Monomere am Eingang eines ersten Extruders eingeleitet werden und das Polymerisat, gegebenenfalls als Formkörper, am Ende eines Extruders ausgegeben wird.